(19) Europäisches Patentamt
European Patent Office
Office européen des brevets

(11) Numéro de publication: **0 123 605**
**B1**

# (12) FASCICULE DE BREVET EUROPÉEN

(45) Date de publication du fascicule du brevet: **05.11.86**

(21) Numéro de dépôt: **84400718.7**

(22) Date de dépôt: **11.04.84**

(51) Int. Cl.[4]: **C 07 D 253/06**, C 07 D 409/04, C 07 D 407/04, C 07 D 401/04, A 61 K 31/53

(54) **N-Cyclopropylméthyl-2 oxo-3 diparaméthoxyphenyl 5-6 as triazine, son procédé de fabrication et son utilisation en tant que médicament.**

(30) Priorité: **14.04.83 FR 8306107**

(43) Date de publication de la demande: **31.10.84 Bulletin 84/44**

(45) Mention de la délivrance du brevet: **05.11.86 Bulletin 86/45**

(84) Etats contractants désignés: **AT BE CH DE GB IT LI LU NL SE**

(56) Documents cité:
**FR-A-2 368 278**
**FR-A-2 383 176**
**FR-A-2 500 830**

**A. Burger, Medicinal Chemistry, 3rd edition, part I, p. 77**

(73) Titulaire: **PIERRE FABRE S.A., 125, rue de la Faisanderie, F-75116 Paris (FR)**

(72) Inventeur: **Pitet, Guy, 3, rue de l'Aubisque, F-31000 Toulouse (FR)**
Inventeur: **Cousse, Henri, La Foun de los Nobios Chemin de Lastinos, F-81100 Castres (FR)**
Inventeur: **Mouzin, Gilbert, 21, rue Sainte- Foy, F-81100 Castres (FR)**

(74) Mandataire: **Ahner, Francis, CABINET REGIMBEAU 26, avenue Kléber, F-75116 Paris (FR)**

**EP 0 123 605 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention, réalisée au Centre de Recherches PIERRE FABRE, a pour objet la N-cyclopropylméthyl-2 oxo-3 diparaméthoxyphényl 5-6 as triazine de formule I

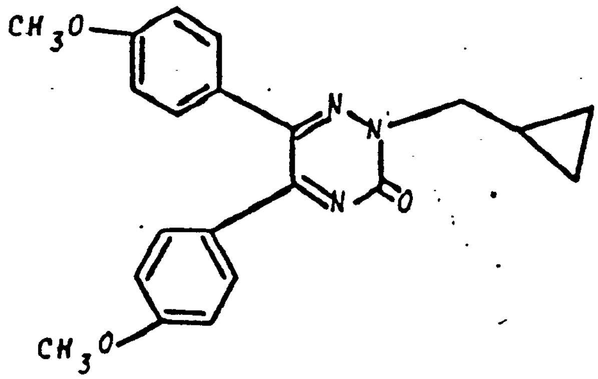

(I)

son procédé de fabrication et son utilisation en tant que médicament.

Dans la technique antérieure, par exemple illustrée par les brevets FR-A- n° 2 368 278, n° 2 383 176, n° 2 478 095 et n° 2 500 830 déposés au nom de la demanderesse, il a été démontré que les composés as triaziniques possédaient des propriétés antalgiques, lorsqu'ils étaient substitués en position 2 sur l'azote par les groupes:

. alcoyle, aryle et alcényle (FR-A- n° 2 368 278)

. alcynyle et amino alcoyle (FR-A- n° 2 383 176)

. cétoniques et plus particulièrement alcoyl C=O alcoyle et alcoyl -C=O aryle (FR-A- n° 2 478 095)

. morpholino alcoyle, diméthylaminoéthyle, alcoxy alcoyle, alcénoxy alcoyle, hydroxyalcoyle, acétamido, halogénoalcoyle, diméthylalcoyle, N-pipérazino alcoyle, diéthyl malonyl-2, alcénylamino alcoyle, nicotinoyloxy éthyl-2 et dihydroxy alcoyle (FR-A- n° 2 500 830).

Certains de ces produits avaient en outre une composante antiagrégante intéressante in vitro.

La poursuite des investigations sur cette structure as triazinique a permis de conduire au nouveau dérivé objet de la présente invention.

Ce composé se démarque des brevets antérieurs de la demanderesse par la nature cycloalcoylique du substituant en position 2 sur l'azote.

Il a en effet été constaté de façon tout à fait inattendue que la présence de ce groupe cyclopropylméthyl conférait à la molécule une très forte activité antalgique et augmentait d'une façon surprenante la durée d'action du produit ($DE_{50}$ Writhing test PBQ 6 h: - 34 %, alors que pour le meilleur produit des brevets précédents l'activité antalgique était nulle après 2 heures).

Enfin et surtout, l'activité de ce nouveau dérivé apparaît chez l'animal très rapidement.

La N-cyclopropyl méthyl-2 oxo-3 diparaméthoxyphényl 5-6 as triazine a une très forte activité antiagrégante.

Inhibition in vitro de la MDA plaquettaire:

$IC_{50} = 5 \times 10^{-7} M$

alors que le composé le plus actif revendiqué dans les brevets précédents est:

$IC_{50} = 5 \times 10^{-5} M$.

De plus, le nouveau composé selon l'invention est actif ex vivo et in vivo contrairement aux composés de la technique antérieure.

La présente invention concerne également la préparation du nouveau dérivé de formule I.

Selon l'invention, ce dérivé est obtenu en traitant la oxo-3 diparaméthoxypheényl 5-6 as triazine de formule II

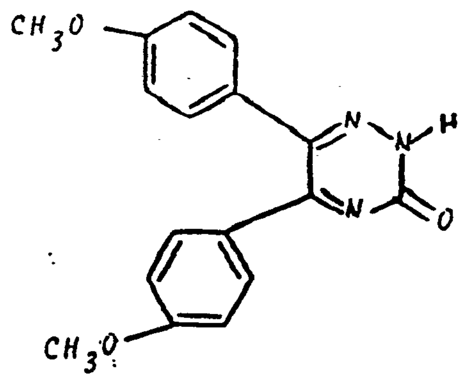

(II)

par un agent sodant au sein d'un solvant organique, puis en condensant un halogénure de cyclopropylméthyl de formule générale III

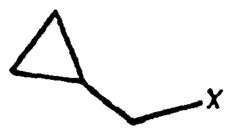

(III)

dans laquelle:

X représente un atome d'halogène.

Dans la pratique, l'agent sodant utilisé sera généralement un hydrure de métal alcalin et plus particulièrement l'hydrure de sodium.

L'oxo-3 diaryl 5-6 as triazine de départ peut être synthétisée par exemple selon la méthode décrite dans les brevets précités en condensant la dicétone appropriée sur la semicarbazide:

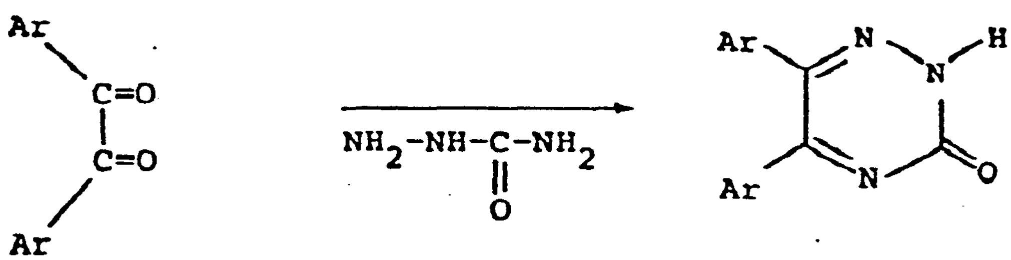

L'invention concerne enfin l'utilisation de la N-cyclopropylméthyl-2 oxo-3 diparaméthoxyphényl 5-6 as triazine en tant que médicament utile dans le traitement des algies rebelles et des migraines, ainsi que les compositions pharmaceutiques la renfermant à titre de principe actif.

L'invention sera décrite ci-après plus en détail à propos d'un exemple non limitatif de préparation:

**Exemple 1**

**Préparation de la N-cyclopropylméthyl-2 oxo-3 diparaméthoxy phényl 5-6 as triazine**

**1) Préparation du bromométhylcyclopropane Réactifs**

25 g (0,345 m) de cyclopropylcarbinol
12 ml (0,125 m) de $PBr_3$
110 ml d'éther éthylique

**Mode opératoire**

On refroidit à -70° C 25g de cyclopropylcarbinol dans 100 ml d'éther, puis on ajoute 12 ml de $PBr_3$ en solution dans 110 ml d'éther éthylique.

On laisse revenir à température ambiante et on laisse sous forte agitation 30 minutes.

On additionne 10 ml d'eau, on décante, lave à l'eau et sèche sur sulfate de sodium.

On évapore lentement l'éther éthylique à pression atmosphérique. On récupère avec un rendement quantitatif le bromométhylcyclopropane. Ce produit a une pureté > à 90 % (dosage CPV) et il est utilisé brut pour l'étape suivante.

**2) Préparation de la N-cyclopropylméthyl-2 oxo-3 diparaméthoxyphényl 5-6 as triazine**

**Réactifs**

56,15 g (0,18 mole) d'oxo-3 diparaméthoxyphényl 5-6 as triazine
36,15 g (0,27 mole) de bromométhyl-2-cyclopropane
7,76 g (0,27 mole) d'hydrure de sodium à 80 %
900 ml de diméthylacétamide anhydre.

**Mode opératoire**

Dans un ballon de 3 litres muni d'une agitation mécanique, on introduit 7,76 g d'hydrure de sodium à 80 % (préalablement lavé à l'éther éthylique anhydre). On ajoute 900 ml de diméthylacétamide anhydre et 56,15 g d'oxo-3 diparaméthoxyphényl 5-6 as triazine.

On agite une nuit à température ambiante, puis on introduit 36,15 g de bromométhyl-2-cyclopropane.

Après 30 minutes d'agitation, le mélange réactionnel devient limpide.

On maintient deux heures l'agitation à température ambiante, puis on introduit goutte à goutte 30 ml d'eau pour détruire l'excès d'hydrure de sodium.

On évapore sous pression réduite à 70° C le diméthylacétamide, l'huile résiduelle obtenue est laissée une nuit au réfrigérateur, elle cristallise lentement.

On reprend les cristaux bruts par de l'acétate d'éthyle, on lave la phase organique à l'eau, puis on sèche sur sulfate de sodium et on filtre.

On évapore jusqu'à siccité l'acétate d'éthyle, l'huile résiduelle cristallise rapidement.

On triture les cristaux dans 300 ml d'éther éthylique, on glace et on filtre. On récupère avec un rendement de 74 %, 48 g de produit de formule:

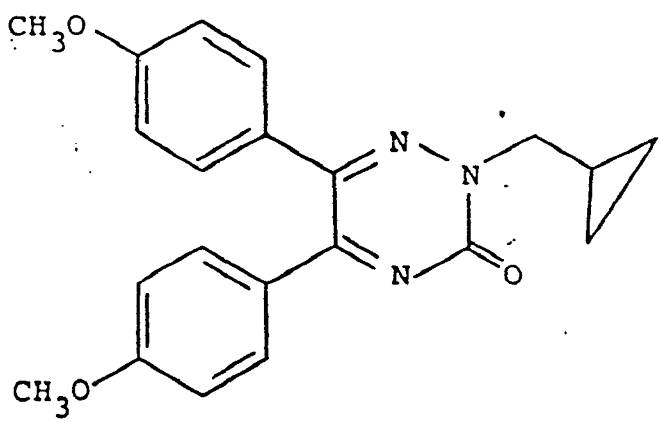

Formule brute: $C_{21}H_{21}N_3O_3$
Masse moléculaire: 363,42
Cristaux: jaune clair
Point de fusion: 114° C
Chromatographie sur plaque:
- support: gel de silice 60 F 254 Merck
- solvant: acétate d'éthyle-hexane 50/50
- révélation: UV et iode
- Rf: 0,37

Spectre IR (KBr): ν $cm^{-1}$
1670 (C=O); 1610 (C=C aromatique)
Spectre RMN ($CDCl_3$) δ ppm:
0,5 à 0,8 (m, 4H, $CH_2$ cyclopropanes); 1,5 (m, 1H, CH-cyclopropanes); 3,8 (s, 6H, $-OCH_3$); 4 et 4,1 (d, 2H, $CH_2$-N); 6,7 à 7,6 (m, 8H, aromatiques)
Spectre UV (éthanol 95° GL):
λ max = 246 nm - $\varepsilon_M$ = 18700
Analyse élémentaire:

| | C | H | N |
|---|---|---|---|
| % calculés | 69,40 | 5,82 | 11,56 |
| % trouvés | 69,30 | 5,70 | 11,57 |

**Exprérimentations**

la N-cyclopropylmhéthyl-2 oxo-3 diparaméthoxyhényl 5-6 as triazine a fait l'objet d'essais toxicologiques, pharmacologiques et cliniques qui ont permis de mettre en évidence de remarquables propriétés antalgiques et antiagrégantes.

**a) Toxicologie**

L'étude de la toxicité a été effectuée chez la souris conventionnelle pesant environ 20 grammes.

La substance a été administrée par voie orale et intrapéritonéale. Les doses létales 50 ont été calculées selon la méthode de MILLER et TAINTER - Proc. Soc. Exper. Biol. Med. 1944, 57, 261. Elles sont respectivement > à 1500 mg/kg par voie orale et > à 500 mg/kg par voie I.P.

**b) Propriétés pharmacologiques**

**1) Propriétés analgésigues**

L'activité sur le test des contorsions à la phényl benzoquinone (selon SIEGMUND et coll. - J. Pharm. Expt. Ther. 1957, 119,453) a été déterminée après administration du produit per os.

La $DE_{50}$ du produit est de 1,4 mg/kg.

La cinétique d'action du produit à 3 mg/kg per os est rapportée dans le tableau suivant:

| temps | % de diminution des contorsions |
|---|---|
| 5 min. | - 78 % |
| 30 min. | - 62 % |
| 1 h | - 41 % |
| 2 h | - 35 % |
| 6 h | - 34 % |

**2) Propriétés antiagrégantes**

- <u>in vitro</u>

Sur le test d'inhibition in vitro à la MDA plaquettaire le produit présente une $IC_{50}$ de $5.10^{-7}$M.

- <u>ex vivo</u>

Sur ce test pratiqué ex vivo le produit présente une $ED_{50}$ de 1,5 mg/kg.

- <u>in vivo</u>

Sur le test de 4 jours, le produit provoque une inhibition totale vis-à-vis de l'acide arachidonique à la dose de $5.10_{-6}$M.

**c) Applications thérapeutiques**

La N-cyclopropylméthyl-2 oxo-3 diparaméthoxyphényl 5-6 as triazine est faiblement colorée, elle est nettement mieux tolérée par administration répétée que les molécules décrites dans la technique antérieure.

Compte tenu de sa parfaite tolérance et de ses remarquables propriétés pharmacologiques, le composé chimique objet de l'invention peut être utilisé, dans le traitement des algies rebelles justiciables de traitement prolongé.

Les résultats se sont avérés également satisfaisants dans le cas du traitement des troubles migraineux.

Les préparations pharmaceutiques contenant ce principe actif peuvent être administrées par voie orale, parentérale ou rectale. Ces compositions pharmaceutiques peuvent également contenir, en association, d'autres principes actifs pharmaceutiquement et thérapeutiquement acceptables.

La dose par unité de prise sera par exemple comprise entre environ 10 et environ 100 mg.

Les quelques exemples suivants de préparations pharmaceutiques contenant le principe actif objet de la présente invention, sont donnés à simple titre indicatif.

**a) Comerimés**

N-cyclopropylméthyl-2 oxo-3 diparaméthoxyphényl 5-6 as triazine... 15 mg

Excipient: lactose

**b) Gélules**

N-cyclopropylméthyl-2 oxo-3 diparaméthoxyphényl 5-6 as triazine... 30 mg

**c) Suppositoires adultes**

N-cyclopropylméthyl-2 oxo-3 diparaméthoxyphényl 5-6 as triazine.............. 50 mg

Glycérides semi-synthétiques q.s.p.... 1 suppositoire de lg.

**Revendications**

1) N-cyclopropyl-méthyl-2 oxo-3 diparaméthoxyphényl 5-6 as triazine de formule I

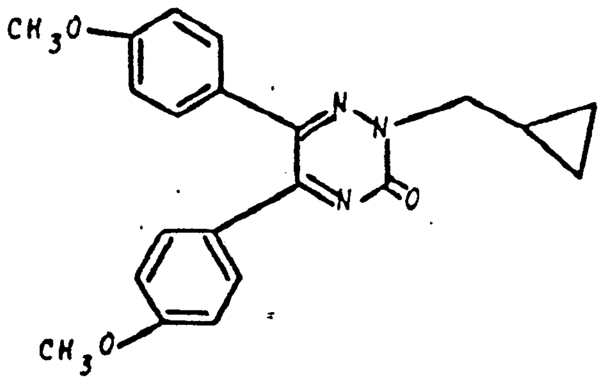

(I)

2) Procédé de préparation du composé de formule I, caractérisé en ce que l'on traite l'oxo-3 diparaméthoxyphényl 5-6 as triazine de formule II

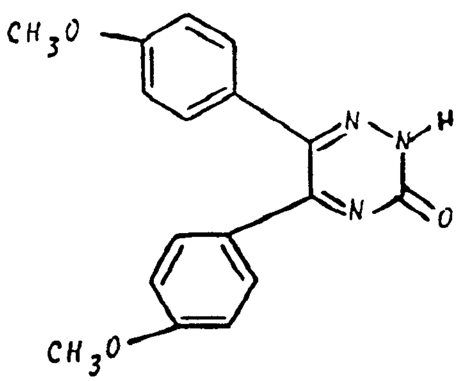

(II)

par un agent sodant au sein d'un solvant organique, puis que l'on condense un halogénure de méthylcyclopropane de formule III

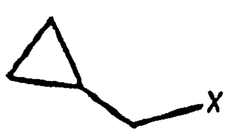

(III)

dans laquelle X représente un atome d'halogène.

3) Procédé de préparation selon la revendication 2, caractérisé en ce que l'agent sodant utilisé est un hydrure de métal alcalin.

4) Procédé de préparation selon la revendication 3, caractérisé en ce que l'hydrure de métal alcalin est

l'hydrure de sodium.

5) Procédé de préparation selon l'une des revendications 2 et 4, caractérisé en ce que le solvant utilisé est le diméthylacétamide.

6) Procédé selon l'une des revendications 2 à 5, caractérisé en ce que la sodation et la condensation de l'halogénure de méthylcyclopropane sont effectuées à température ambiante.

7) Procédé selon l'une des revendications 2 à 6, caractérisé en ce que l'halogénure de méthylpyclopropane est le bromométhyl-2-cyclopropane.

8) A titre de médicament, la N-cyclopropyl-méthyl-2 oxo-3 diparaméthoxyphényl 5-6 as triazine.

9) Compositions pharmaceutiques, caractérisées en ce qu'elles contiennent à titre de principe actif la N-cyclopropylméthyl-2 oxo-3 diparaméthoxyphényl 5-6 as triazine.

**Patentansprüche**

1. 2-N-Cyclopropylmethyl-3-oxo-5,6 -di-p-methoxyphenyl-as-triazin der Formel (I)

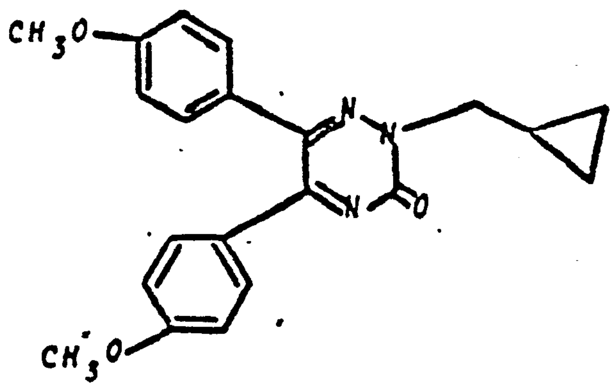

(I)

2. Verfahren zum Herstellen der Verbindung der Formel (I), dadurch gekennzeichnet, daß man 3-Oxo-5,6-di-p-methoxyphenyl-as-triazin der Formel (II)

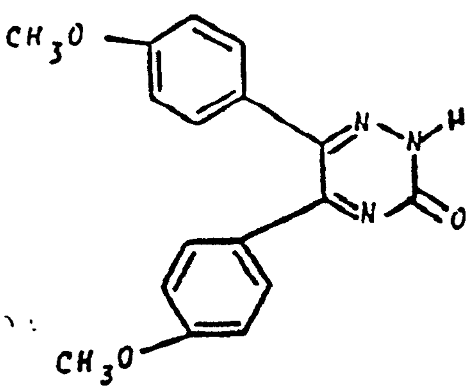

(II)

in einem organischen Lösungsmittel mit einem metallsalzbildenden Mittel behandelt und daß man dann mit einem Methylcyclopropanhalogenid der Formel (III)

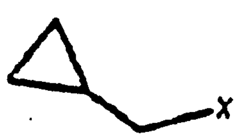

(III)

worin X ein Halogenatom darstellt, kondensiert.

3. Herstellungsverfahren nach Anspruch 2, dadurch gekennzeichnet, daß das verwendende metallsalzbildende Mittel ein Alkalimetallhydrid ist.

4. Herstellungsverfahren nach Anspruch 3, dadurch gekennzeichnet, daß das Alkalimetallhydrid Natriumhydrid ist.

5. Herstellungsverfahren nach einem der Ansprüche 2 und 4, dadurch gekennzeichnet, daß das verwendete Lösungsmittel Dimethylacetamid ist.

6. Verfahren nach einem der Ansprüche 2 bis 5, dadurch gekennzeichnet, daß die Metallsalzbildung und die Kondensation des Methylcyclopropanhalogenids bei Umgebungstemperatur durchgeführt werden.

7. Verfahren nach einem der Ansprüche 2 bls 6, dadurch gekennzeichnet, daß das Methylcyclopropanhalogenid 2-Brom-methyl-cyclopropan ist.

8. Als Heilmittel das 2-N-Cyclopropylmethyl-3-oxo-5,6-di-p-methoxyphenyl-as-triazin.

9. Pharmazeutische Mischungen, dadurch gekennzeichnet, daß sie als Wirkstoff das 2-N-Cyclopropylmethyl-3-oxo-5,6,-di-p-methoxyphenyl-as-triazin enthalten.

**Claims**

1) N-cyclopropyl-methyl-2-oxo-3 diparamethoxyphenyl 5-6 as triazine of formula 1

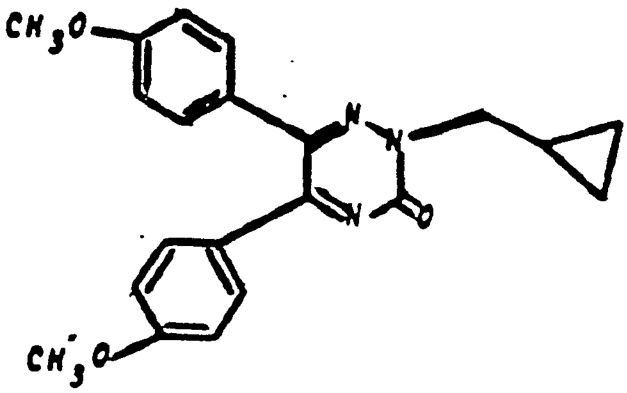

(I)

2) A method of preparation of the compound of formula 1, characterised in that the oxo-3 diparamethoxyphenyl 5-6 is treated as triazine of formula II

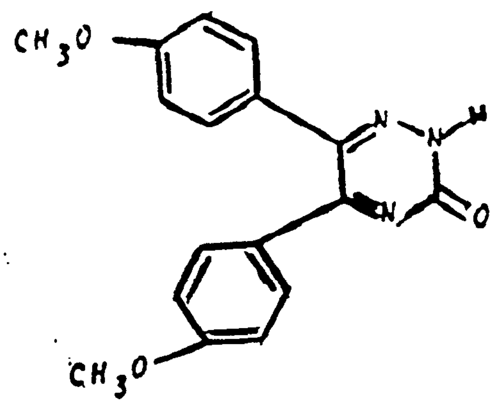

(II)

with a sodium-providing agent within an organic solvent, then in that condensation is effected with a methylcyclopropane halide of formula III

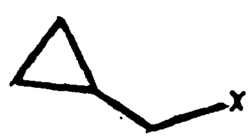

(III)

wherein X is a halogen atom.

3) A method of preparation according to Claim 2, characterised in that the sodium-providing agent used is an alkali metal hydride.

4) A method of preparation according to Claim 3, characterised in that the alkali metal hydride is sodium hydride.

5) A method of preparation according to one of Claims 2 and 4, characterised in that the solvent used is

dimethylacetamide.

6) A method according to one of Claims 2 to 5, characterised in that provision of sodium to, and condensation of, the methylcyclopropane halide are carried out at ambient temperature.

7) A method according to one of Claims 2 to 6, characterised in that the methycyclopropane halide is bromomethyl-2-cyclopropane.

8) As a drug, N-cyclopropyl-methyl-2-oxo-3 diparamethoxyphenyl 5-6 as triazine.

9) Pharmaceutical compositions, characterised in that they contain as the active principle N-cyclopropylmethyl-2 oxo-3 diparamethoxyphenyl 5-6 as triazine.